(19) Europäisches Patentamt
European Patent Office
Office européen des brevets

(11) **EP 4 125 558 B1**

(12) **EUROPEAN PATENT SPECIFICATION**

(45) Date of publication and mention
of the grant of the patent:
**31.01.2024 Bulletin 2024/05**

(21) Application number: **21721253.9**

(22) Date of filing: **30.03.2021**

(51) International Patent Classification (IPC):
**A61B 5/00** *(2006.01)*

(52) Cooperative Patent Classification (CPC):
**A61B 5/0095; A61B 5/7264**

(86) International application number:
**PCT/US2021/024761**

(87) International publication number:
**WO 2021/202438 (07.10.2021 Gazette 2021/40)**

(54) **SYSTEM, METHOD, AND APPARATUS FOR MULTI-SPECTRAL PHOTOACOUSTIC IMAGING**

SYSTEM, VERFAHREN UND VORRICHTUNG ZUR MULTISPEKTRALEN FOTOAKUSTISCHEN BILDGEBUNG

SYSTÈME, PROCÉDÉ ET APPAREIL D'IMAGERIE PHOTOACOUSTIQUE MULTISPECTRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2020 US 202063002714 P**

(43) Date of publication of application:
**08.02.2023 Bulletin 2023/06**

(73) Proprietor: **FUJIFILM SonoSite, Inc.**
**Bothell, WA 98021 (US)**

(72) Inventors:
• **GRASSO, Valeria**
**1098 XB Amsterdam (NL)**
• **JOSE, Jithin**
**Manchester M22 4UD (GB)**

(74) Representative: **Dehns**
**St. Bride's House**
**10 Salisbury Square**
**London EC4Y 8JD (GB)**

(56) References cited:
**US-A1- 2015 101 411**

• **ARABUL M.U. ET AL: "Unmixing multi-spectral photoacoustic sources in human carotid plaques using non-negative independent component analysis", PHOTOACOUSTICS, [Online] vol. 15, 1 September 2019 (2019-09-01), page 100140, XP055810980, ISSN: 2213-5979, DOI: 10.1016/j.pacs.2019.100140 Retrieved from the Internet: URL:https://pure.tue.nl/ws/files/138754036 /1_s2.0_S2213597918300260_main.pdf> [retrieved on 2021-06-07]**
• **Glatz Urgen ET AL: "Blind source unmixing in multi-spectral optoacoustic tomography References and links", Nat. Biotechnol. Rev. Sci. Instrum. Nat. Methods Opt. Lett. Phys. Rev. Lett. Chem. Rev. Opt. Express Opt. Lett. Appl. Phys. Lett. Clin. Chem. J. Biomed. Opt. OPTICS EXPRESS Opt. Lett. Nat. Photonics Opt. Express Med. Phys. IEEE Trans. Med. Imaging An, 1 January 2011 (2011-01-01), pages 848-851, XP055810988, Retrieved from the Internet: URL:https://www.researchgate.net/publicati on/50268021_Blind_source_unmixing_in_multi -spectral_optoacoustic_tomography [retrieved on 2021-06-07]**

**EP 4 125 558 B1**

- **GRASSO VALERIA ET AL: "An Automatic Unmixing Approach to Detect Tissue Chromophores from Multispectral Photoacoustic Imaging", SENSORS, [Online] vol. 20, no. 11, 6 June 2020 (2020-06-06), page 3235, XP055810939, DOI: 10.3390/s20113235 Retrieved from the Internet: URL:https://www.ncbi.nlm.nih.gov/pmc/artic les/PMC7308815/pdf/sensors-20-03235.pdf> [retrieved on 2021-06-07]**

**Description**

**BACKGROUND**

The practice of observing tissue chromophores can be a helpful tool in the early detection, prediction, and monitoring of various diseases or health conditions. Molecular imaging can be one of the methods used to detect and quantify tissue chromophores. In particular, multi-spectral photoacoustic (PA) imaging has emerged as a useful, non-invasive molecular imaging tool to visualize tissue chromophores. The underlying principle of PA imaging is using nanosecond laser pulses to illuminate different wavelengths into the biological tissue. Depending on the optical absorption coefficient of the tissue chromophores, the tissue can absorb the laser light and produce acoustic waves caused by thermo-elastic expansion of the tissue. Similar to conventional ultrasound imaging, these generated photoacoustic signals can be detected and the optical absorption of the tissue chromophores can be reconstructed. PA imaging is therefore considered a hybrid imaging modality that combines the optical absorption contrast of the chromophores and the spatial acoustic resolution and penetration depth of ultrasound imaging to provide molecular information.

Being a hybrid imaging modality of ultrasound and optical, this multi-modal imaging technology can provide anatomical, functional, and molecular information several centimeters deep in the tissues with a resolution up to tens of micrometers. PA imaging can be used in various preclinical applications, such as tumor progression, prediction of tumor recurrence, therapy monitoring, imaging of vasculature, and the bio distribution of contrast agents. In addition to preclinical applications, PA imaging can be used in clinical trials or applications. For example, breast cancer imaging, sentinel lymph node imaging, and/or examining temporal arteries in patients with suspect giant-cell arteritis (GCA). Other usages have included using PA imaging in low-resource settings for the visualization of superficial vasculatures and needle guidance for minimally invasive procedures.

While emphasis has generally been placed on hardware development of PA imaging, the data analysis and reconstruction algorithms can play an important role in increasing the sensitivity of PA imaging.

Known PA imaging systems are disclosed in the following publications:

- ARABUL M.U. ET AL: "Unmixing multi-spectral photoacoustic sources in human carotid plaques using non-negative independent component analysis", PHOTOACOUSTICS, vol. 15 1 September 2019;
- Glatz Urgen ET AL: "Blind source unmixing in multi-spectral optoacoustic tomography References and links", Nat. Biotechnol. Rev. Sci. Instrum. Nat. Methods Opt. Lett. Phys. Rev. Lett. Chem. Rev. Opt. Express Opt. Lett. Appl. Phys. Lett. Clin. Chem. J. Biomed. Opt. OPTICS EXPRESS Opt. Lett. Nat. Photonics Opt. Express Med. Phys. IEEE Trans. Med. Imaging An, 1 January 2011;
- US 2015/101411 A1.

**SUMMARY**

The disclosed subject matter described below provides for a non-limiting example of an improved PA imaging system, apparatus, and method. For example, embodiments provide an unsupervised spectral unmixing process or algorithm for processing PA image data. Using the unsupervised spectral unmixing process or algorithm can help to improve the detection of tissue chromophores, thereby improving the monitoring, diagnosis, or treatment of a disease or medical condition associated with the detected tissue chromophores.

According to a first aspect, the invention provides a photoacoustic imaging method including receiving multi-spectral photoacoustic image data from a photoacoustic (PA) imaging system. The method also includes pre-processing the multi-spectral photoacoustic image data. The pre-processing includes determining a number of significant components above a noise floor of the multi-spectral photoacoustic image data. In other words, the noise floor or level can be used to define the number of significant components from the multispectral photoacoustic image data. In addition, the method includes detecting tissue chromophores based on the number of significant components from the multi-spectral photoacoustic image data using an unsupervised spectral unmixing process. The unsupervised spectral unmixing process includes clustering and windowing of the multi-spectral photoacoustic image data. In other words, the windowing and clustering can be used to determine the prominent tissue chromophores present in the multi-spectral photoacoustic image data. Further, the method includes displaying the detected tissue chromophores in an abundance map.

According to a second aspect, the invention provides a photoacoustic imaging apparatus including at least one memory comprising computer program code, and at least one processor. The at least one memory comprising the computer program code are configured, with the at least one processor, to cause the photoacoustic imaging apparatus at least to perform the method of the first aspect.

According to a third aspect, the invention provides a non-transitory computer-readable medium encoding instructions that, when executed on a photoacoustic imaging apparatus, perform a process according to the first aspect.

According to a fourth aspect, the invention provides a computer program product encoding instructions for performing, when run on a photoacoustic imaging apparatus, a process according to the method of the first aspect.

## BRIEF DESCRIPTION OF THE DRAWINGS

FIG. 1 is a diagram illustrating a supervised unmixing system or method.
FIG. 2 is a diagram illustrating an unsupervised unmixing system.
FIG. 3 is a diagram illustrating exemplary unsupervised unmixing algorithms.
FIG. 4 is a diagram illustrating an unsupervised unmixing system or method.
FIG. 5 is a diagram illustrating pre-processing.
FIG. 6 is a diagram illustrating abundance maps for supervised and unsupervised unmixing algorithms.
FIG. 7 is a diagram illustrating a component spectra.
FIG. 8 is a flow diagram of a method or process.
FIG. 9 is a diagram illustrating exemplary components of a system or apparatus.

## DETAILED DESCRIPTION

Reference will now be made in detail to the various exemplary embodiments, which embodiments are illustrated in the accompanying drawings. The structure and corresponding method of operation of the disclosed subject matter will be described in conjunction with the detailed description of the system. The examples and embodiments described below are merely exemplary, and should not be taken in any way as limiting the scope of the invention. The scope of the invention is defined by the appended claims.

In certain non-limiting embodiments, PA imaging employs spectral unmixing algorithms. In particular, since the optical absorption coefficient of the tissue chromophores varies over the spectrum, multispectral image processing approaches can be applied to to some examples of the disclosed subject matter.

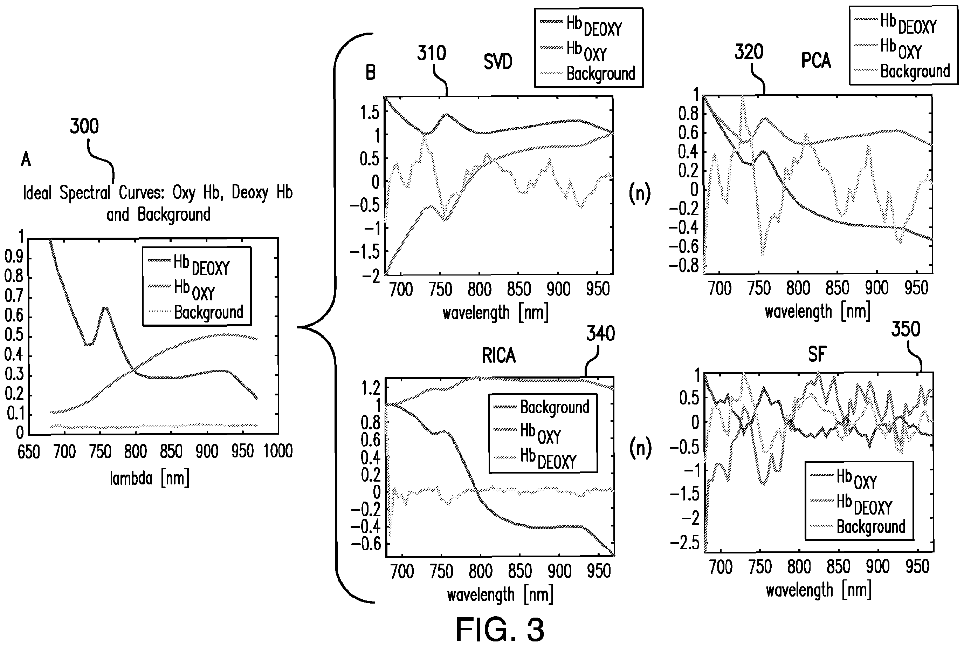

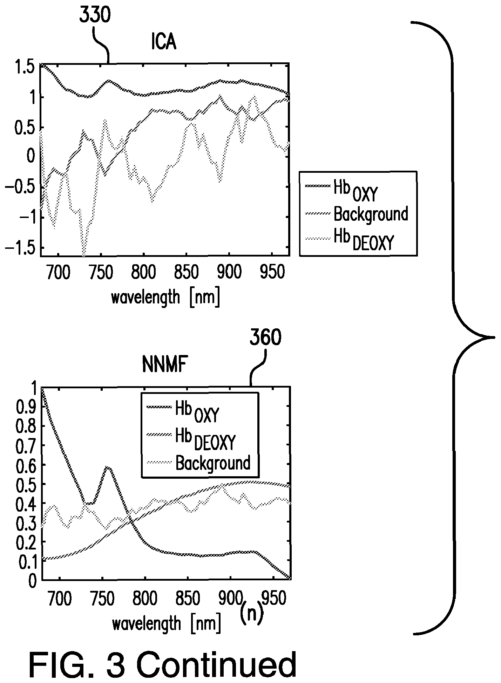

FIG. 3 is a diagram illustrating embodiments of unsupervised unmixing algorithms according to some examples of the disclosed subject matter.

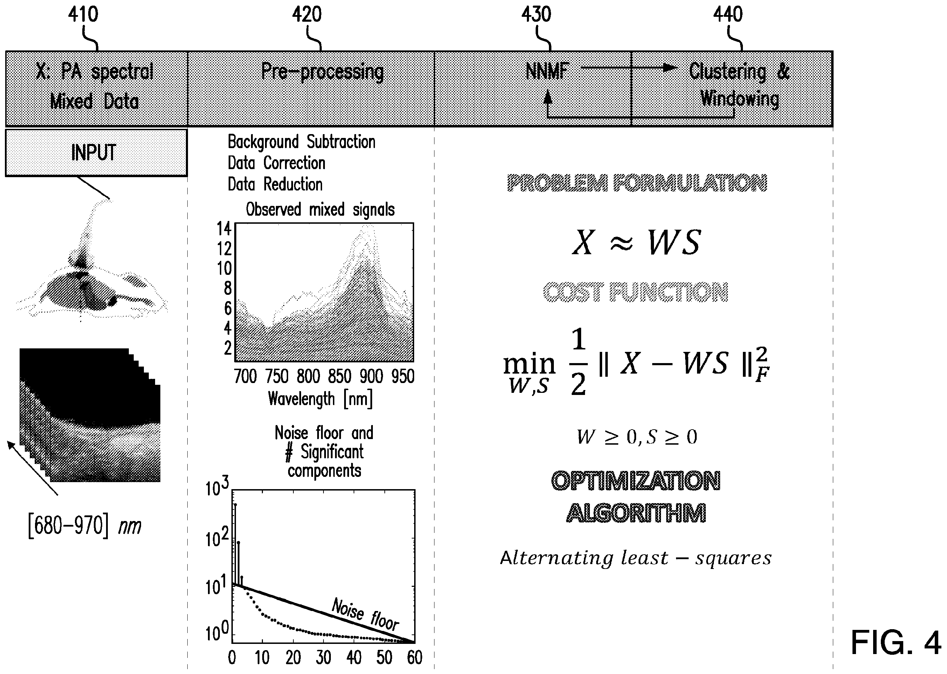

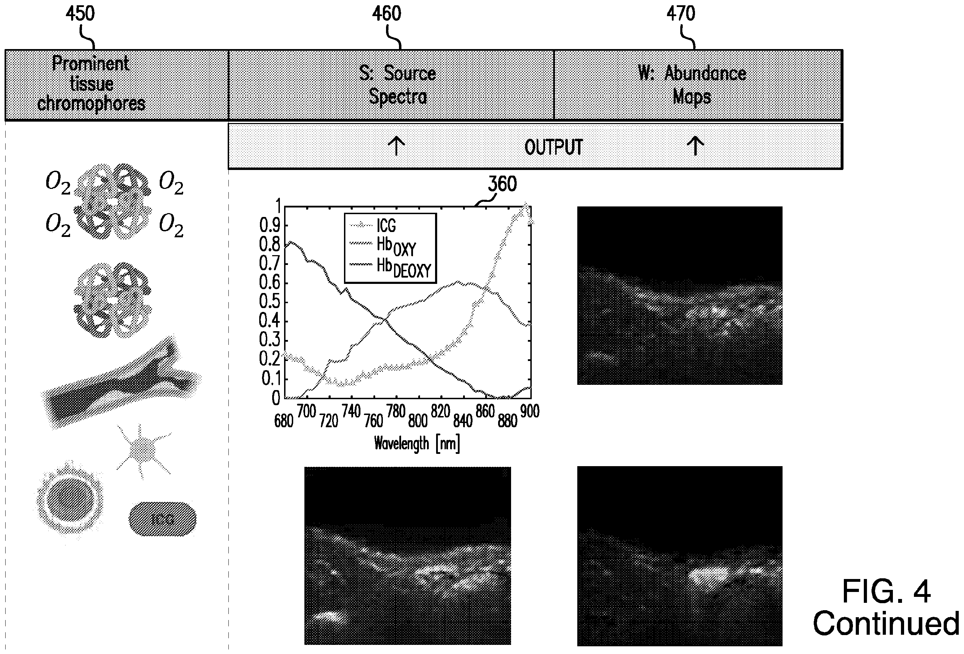

FIG. 4 is a diagram illustrating an unsupervised unmixing system or method according to some examples of the disclosed subject matter.

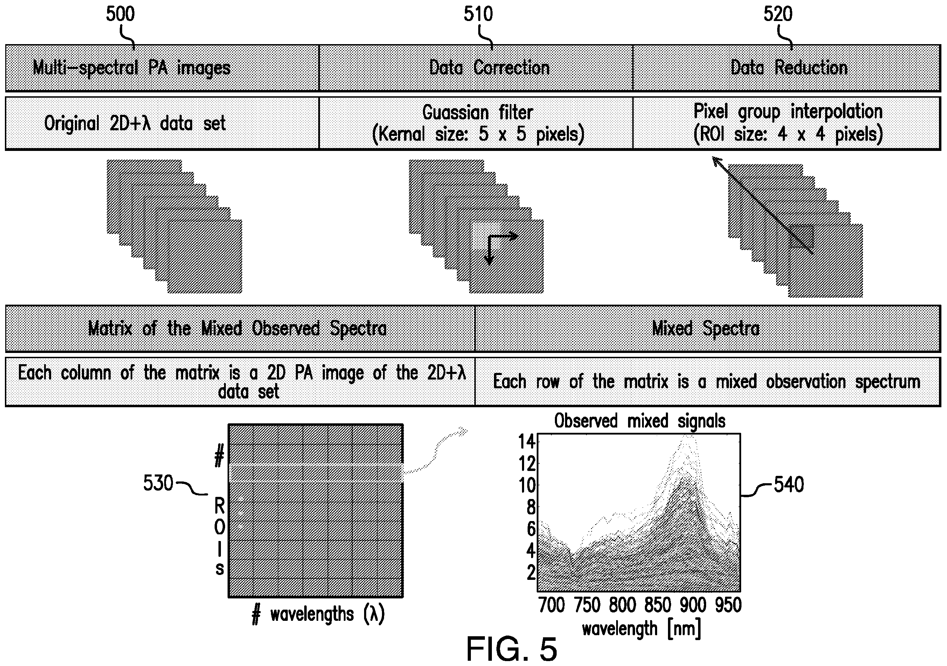

FIG. 5 is a diagram illustrating pre-processing according to some examples of the disclosed subject matter.

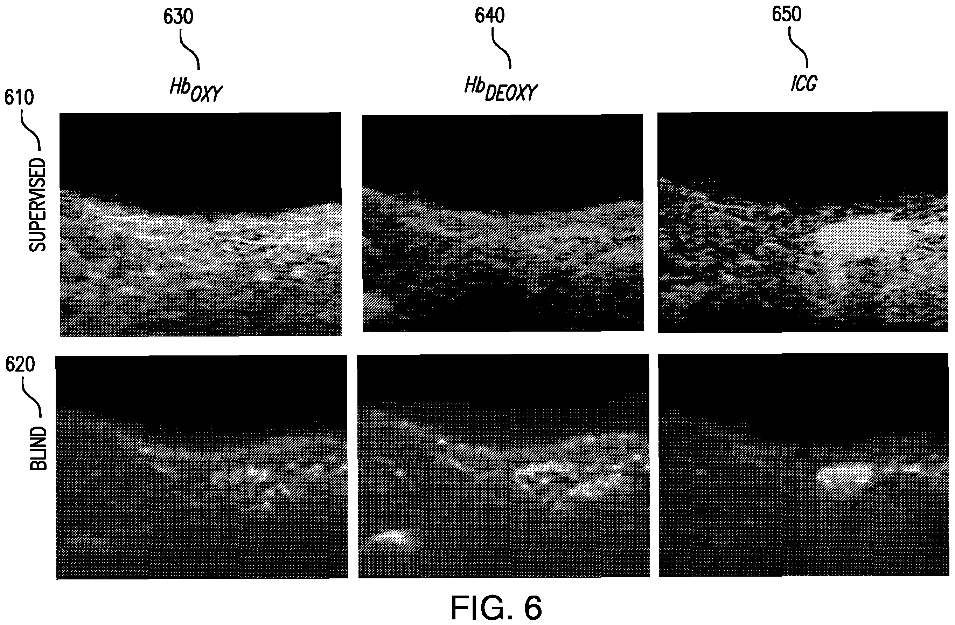

FIG. 6 is a diagram illustrating abundance maps for supervised and unsupervised unmixing algorithms according to some examples of the disclosed subject matter.

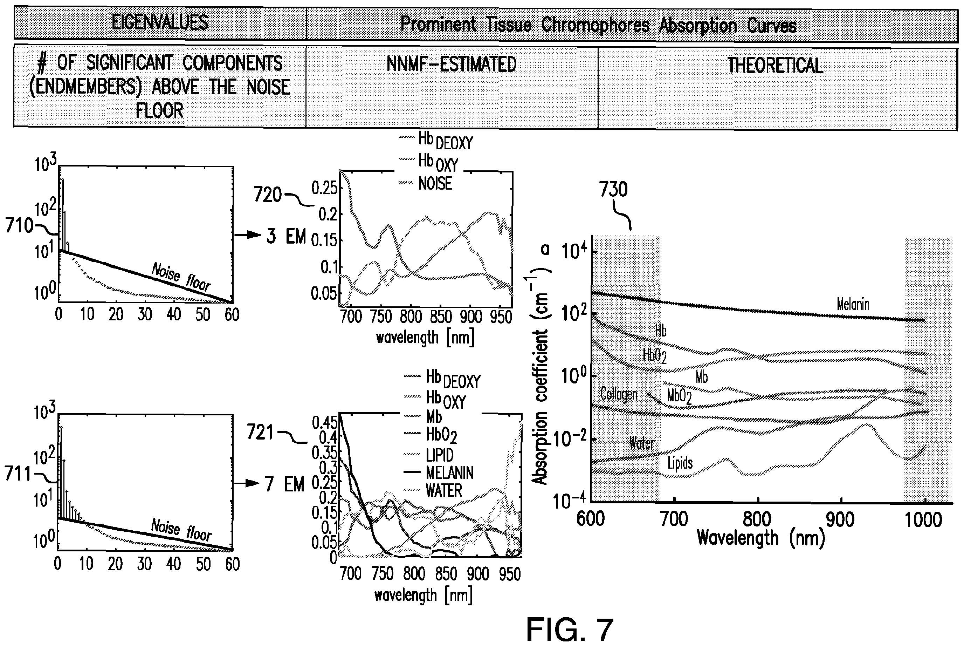

FIG. 7 is a diagram illustrating a component spectra according to some examples of the disclosed subject matter.

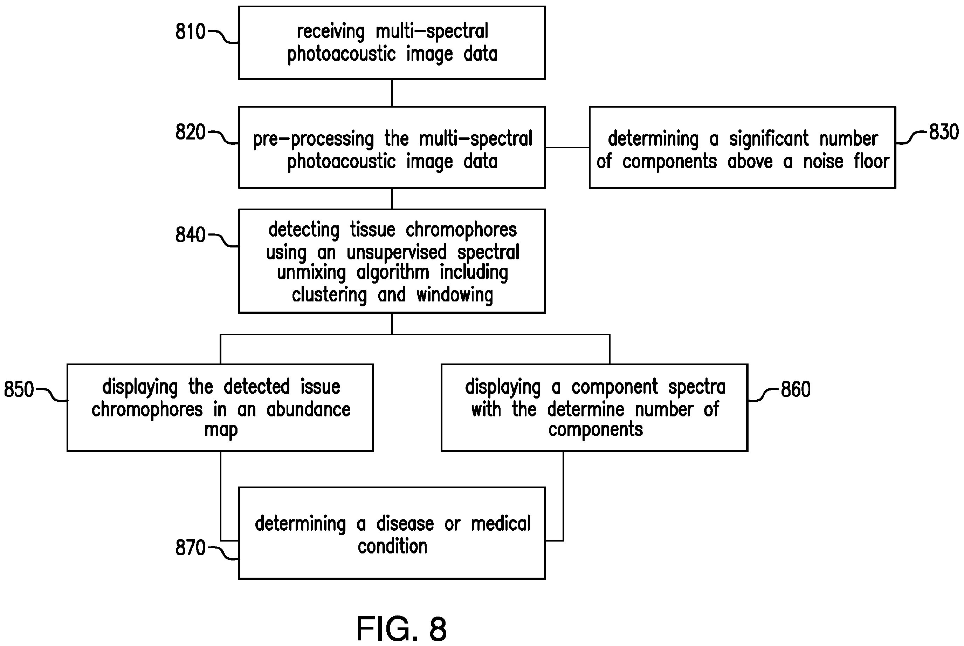

FIG. 8 is a flow diagram of a method or process according to some examples of the disclosed subject matter.

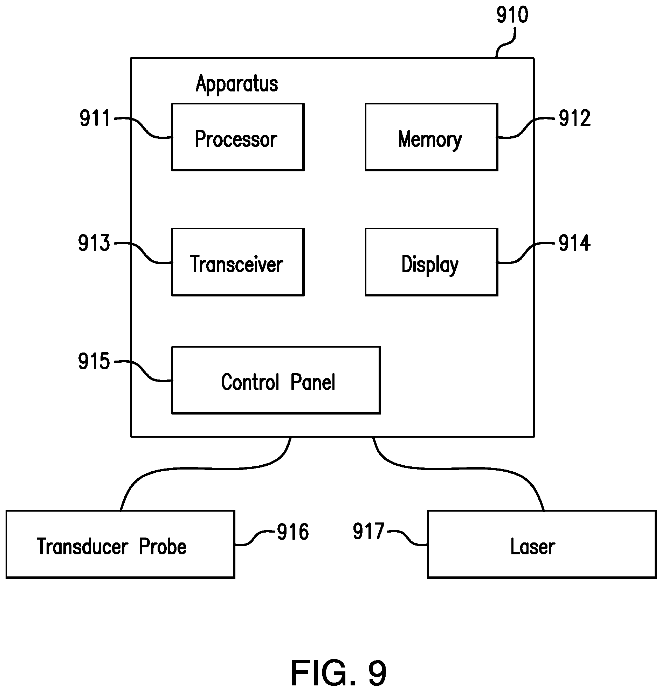

FIG. 9 is a diagram illustrating exemplary components of a system or apparatus according to some examples of the disclosed subject matter.

## DETAILED DESCRIPTION

Reference will now be made in detail to the various exemplary embodiments of the disclosed subject matter, which embodiments are illustrated in the accompanying drawings. The structure and corresponding method of operation of the disclosed subject matter will be described in conjunction with the detailed description of the system. The examples and embodiments described below are merely exemplary, and should not be taken in any way as limiting the scope of the disclosed subject matter.

In certain non-limiting embodiments, PA imaging employs spectral unmixing algorithms. In particular, since the optical absorption coefficient of the tissue chromophores varies over the spectrum, multispectral image processing approaches can be applied to distinguish between chromophores. The pixel intensity of the multi spectral photoacoustic images can be proportional to the absorption value of the respective tissues at a specific wavelength of the light excitation. By considering the pixel size as infinitesimal, every absorption signal throughout the different wavelengths can represent the signal obtained from a single molecular component. However, due to the finite dimension of pixels and the presence of noise, which can corrupt the acquired signal, each spectrum can result in a mixed signal. In some non-limiting embodiments, the mixed signal can be a combination of the absorption spectra of different source chromophores and undesired biological and instrumental noise. A spectral unmixing algorithm can be used to unmix these signals from different optical absorbers and/or to estimate the concentration of a given tissue chromophore.

In particular, spectral unmixing can be a data decomposition approach based on a linear mixing model. To that end, spectral unmixing algorithms can be used to differentiate the mixed pixel spectra into a collection of spectra, also referred to as endmembers, and a set of fractional abundance maps. The component spectra or endmembers represent the pure molecule absorption spectrum extracted from the mixed pixel spectra or the multi-spectral photoacoustic image data. The maps of abundance represent the percentage of each endmember present in a given pixel. The spectral unmixing algorithm can therefore be used as part of the multispectral image processing to characterize molecules present in the tissue based on the spectral absorption profile of a given molecule. In certain non-limiting embodiments the spectral unmixing algorithm can process the received multi-spectral photoacoustic image data to estimate or infer the rest of the image(s).

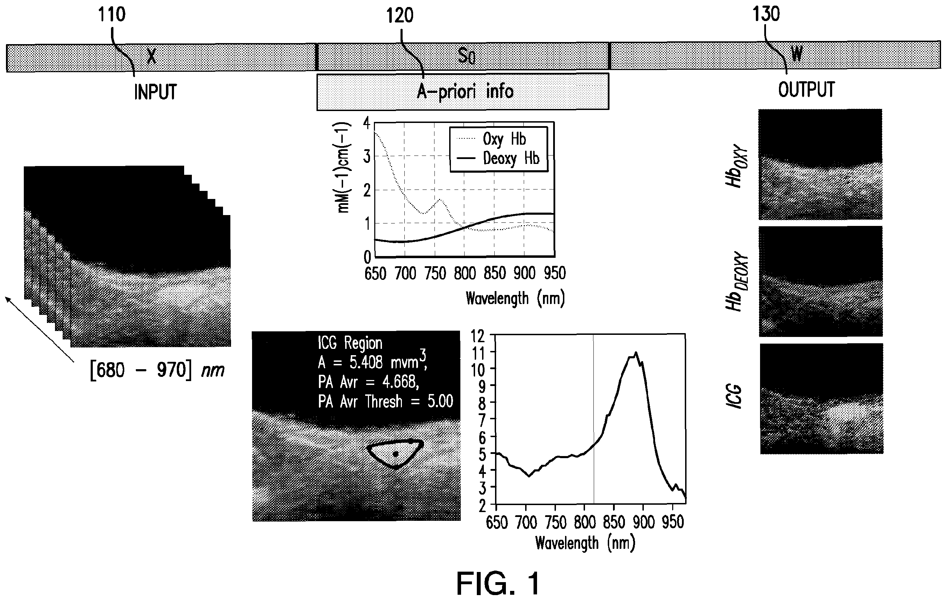

FIG. 1 is a diagram illustrating a supervised unmixing system or method according to some examples of the disclosed subject matter. In particular, FIG. 1 illustrates a method for detecting tissue chromophores with multi-spectral PA imaging using a differential based unmixing algorithm with a known spectral signature as a-prior information. As shown in FIG. 1, input 110 can be a multi-spectral photoacoustic image data obtained from a PA imaging system. The data can be received as images of a two-dimensional region across the wavelength near infrared spectroscopy (NIR) range between 680 and 970 nanometers (nm). In other non-limiting embodiments, any other wavelength range can be used. The multi-spectral photoacoustic image data, for example, can have a step size of 1 nm, 5 nm, or 10 nm. The lower the step size the higher the resolution, meaning that a step size of 1 nm can have a higher resolution than a step size of 5 nm or 10 nm. As such, processing a multi-spectral photoacoustic image data with a step size of 1 nm can require more resources than data having a step size of 5 nm or 10 nm. In some other non-limiting embodiments any other step size can be used. For a wavelength NIR range of 680 - 970 nm with a step size of 5 nm, the data set can include the same or similar two-dimensional region imaged at 59 different wavelengths.

The system or method shown in FIG. 1 can be used to detect the tissue chromophores using a supervised unmixing method. In particular, FIG. 1 uses a differential based unmixing method with a known spectral signature as a-priori information 120. A-priori information 120 can be a user defined endmember absorption spectra of the tissue chromophores, such as an oxyhemoglobin (Oxy Hb) absorption spectrum and deoxyhemoglobin (Deoxy Hb) absorption spectrum. In another example a-priori information can be a predetermined or known region of interest. Based on a-priori information 120 inputted by the user, input 110 can be processed and output 130 can be produced. Output 130 can include one or more abundance maps showing, for example, the distributions of endogenous tissue chromophores like oxyhemoglobin, deoxyhemoglobin and an exogenous contrast agent such as Indocyanine green (ICG). As discussed above, the supervised spectral unmixing approaches can be challenging, as the spectral signatures of the tissues differs with respect to the disease or medical condition. Disease or medical conditions can involve complicated processes that can induce changes in the characteristics of the theoretical absorption spectra of the tissue chromophores. In addition, the absorption spectra of the exogenous contrast agents can also differ due to the interaction, after the injection, within the tissues.

As shown in FIG. 1, some non-limiting embodiments utilize a supervised spectral unmixing algorithm, requiring user interaction. Such supervised algorithms therefore are dependent on a user being able to define a source spectra or a previously identified or known region of interest for prominent chromophores. Because the spectral signature of the tissue differs with respect to diseases or medical conditions, being able to define the source spectra or region of interest can be difficult or biased by the user. The source spectra or the previously identified or known region of interest can be referred to as a-priori information, meaning that a supervising user has to pre-define the information before or during processing. In addition, to obtain an accurate fitting result using a supervised spectral unmixing algorithm, a higher number of wavelengths will be required for the PA image acquisition. Using a higher number of wavelength can increase resource usage by the PA image acquisition system or apparatus, requiring more memory, broadband, network, or processor resources. The supervised spectral unmixing algorithm also relies on user interaction to reduce biological and instrumental noises, both are which are subject to user biases and preferences.

To help overcome one or more of the above disadvantages, certain non-limiting embodiments can utilize one or more unsupervised or autonomous spectral unmixing algorithms. An unsupervised spectral unmixing process or algorithm, which can be a type of unsupervised machine learning algorithm, does not require any user supervision, thereby allowing for automatic detection of prominent component spectra. Prominent component spectra can simply be referred to as components herein. The unsupervised spectral unmixing process or algorithm, for example, can be singular value decomposition (SVD), principal component analysis (PCA), sparse filtering (SF), independent component analysis (ICA), reconstruction independent component analysis (RICA), non-negative matrix factorization (NNMF), or any other unsupervised spectral unmixing process or algorithm known in the art.

In certain non-limiting embodiments, the unsupervised spectral unmixing process or algorithm can be referred to as a blind source separation algorithm. The blind source separation algorithm can be based on iterative optimization methodologies, which includes approximation by minimizing a cost function. One or more cost function can be defined or determined for every blind approach. Each iteration of the algorithm can be used to minimize the cost function. Using this iterative approach, spectral unmixing can help decompose the PA image data into quantitative component maps that identify the biodistribution of the recovered underlying tissue chromophores that provide spectral contrast. The multi-spectral photoacoustic image data inputted into the blind source separation algorithm can be in the form of a single data matrix.

The unsupervised spectral unmixing process or algorithm can allow access to molecular and quantitative information from the PA image data, with high sensitivity and specificity. In some non-limiting embodiments the unsupervised spectral unmixing process or algorithm can allow a system, apparatus, or processor to learn from multi-spectral photoacoustic image data with limited or no human intervention by detecting or recognizing representative spectral patterns. As a data drive method, the unsupervised spectral unmixing process or algorithm can accurately extract hidden underlying features, components, or chromophores.

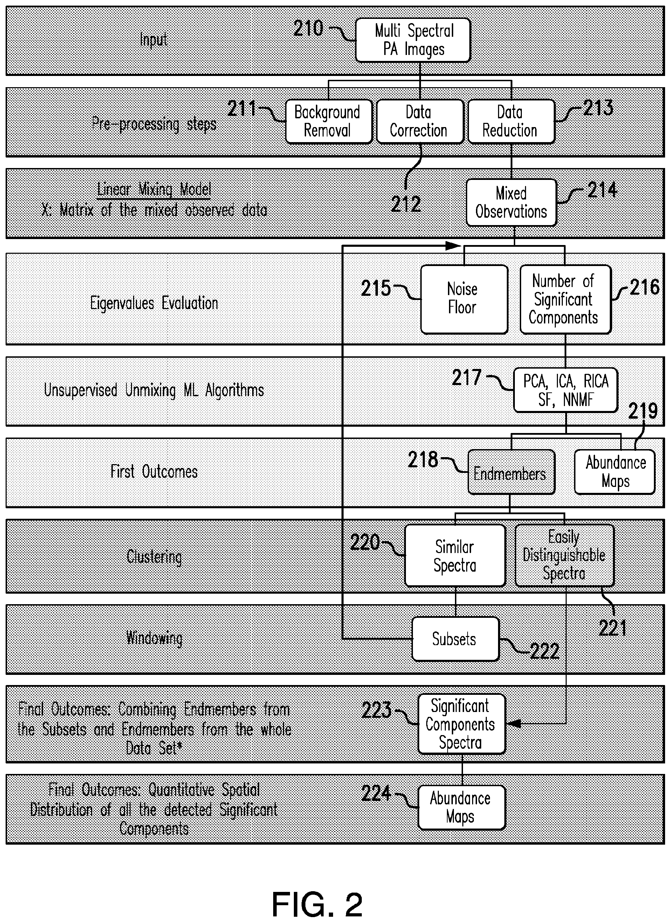

As discussed above, certain non-limiting embodiments can utilize an unsupervised unmixing algorithm based on machine learning that can facilitate the extraction and quantification of the intrinsic absorption trend of biomarkers in both physiological and also in the pathological condition. Using such a data-driven approach, the prominent spectra from the tissue chromophores can be detected from the multi-spectral imaging data set with limited or no user interaction. The resulting tissue chromophores can be visualized and/or quantified with superior sensitivity. FIG. 2 is a diagram illustrating an unsupervised unmixing system or method according to some examples of the disclosed subject matter. In particular, FIG. 2 illustrates the processing of PA image data using an unsupervised mixing algorithm and outputting an abundance map or component spectra.

As shown in FIG. 2, raw multi spectral PA images 210, also referred to as multi-spectral photoacoustic image data, can be inputted to the method, apparatus, or system. Raw multi spectral PA images can be received from a PA imaging machine or device. In some non-limiting embodiments, the ultrasound and PA imaging machine or device can be included within the same machine or device. In certain non-limiting embodiments, raw multi spectral PA images 210 can take the form of a two-dimensional region across part or all of the wavelength NIR range 680 nm - 970 nm. In other non-limiting embodiments, any other wavelength NIR range can be utilized. The step size of the wavelength in the NIR range can be 1 nm, 5 nm, 10 nm, or any other step size. One or more of raw multi spectral PA images 210 can be pre-processed using at least one of background removal 211, data correction 212, and/or data reduction 213. Data correction 212 can include a Gaussian filter to reduce the biological or instrumental noise. The Gaussian filter can process the data using a kernel of 5x5 pixels, or any other sized kernel. Data reduction 213, on the other hand, can include grouping image pixels according to a squared region of interest of 4x4. In other non-limiting embodiments data reduction 213 can include grouping one or more pixels according to a square region of interest, or a region of interest having a different shape, and having one or more pixels.

In certain non-limiting embodiments, the pre-processing can include determining a noise level 215 to define a number of significant components 216 from the multi-spectral photoacoustic image data. The component, for example, can be a biomarker. The unsupervised spectral unmixing process or algorithm 217 can help to facilitate the extraction and quantification of the intrinsic absorption of the one or more biomarkers. For example, the component or biomarker can be melanin, water, collagen, lipids, oxyhemoglobin, deoxyhemoglobin, myoglobin, or any other biomarker known in the art. The detected tissue chromophores can be based on the determined significant components which are above the noise level.

As shown in FIG. 7, for example, significant component spectra which are above the noise floor can be displayed. In some non-limiting embodiments, an eigenvalue algorithm can be used to determine the noise floor and then the components which are significant. The eigenvalue algorithm, for example can be a power iteration, inverse iteration, Rayleigh quotient iteration, locally optimal block preconditioned conjugate gradient algorithm, QR algorithm, Jacobi eigenvalue algorithm, factorable polynomial equations, or any other known equation, method, or algorithm used to determine eigenvalues.

As shown in FIG. 2, after the input and pre-processing step the data can be mixed using a linear mixing model, or any other known mixing model. All or part of the resulting mixed observations 214 can be loaded or inputted to the unsupervised spectral unmixing process or algorithm. Unsupervised spectral unmixing process or algorithm 217 can use an iterative approach to separate pure molecules spectra from the multi-spectral photoacoustic image data. Unsupervised spectral unmixing process or algorithm 217 can include PCA, ICA, RICA, SF, and/or NNMF. For example, NNMF can be used to discriminate the mixed pixel spectra from a multi spectral image into a collection of constituent spectra, also referred to as endmembers 218, and a set of abundance maps 219, which can be referred to fractional abundance maps. The endmembers 218 and/or the set of abundance maps 219 can be referred to as first outcomes. The NNMF can therefore result in a component spectra, spatial distribution maps, and/or abundance maps of the prominent chromophores in the region of interest.

In certain non-limiting embodiments, the unsupervised spectral unmixing process can include clustering and/or windowing. Clustering, for example, can include similar spectra 220 and/or easily distinguishable spectra 221. Windowing, on the other hand, can include dividing the multi-spectral photoacoustic image data into one or more subsets 222. Once one or more subsets 222 are determined, tissue chromophores can be detected for each subset based on the number of significant components using an unsupervised spectra unmixing process. The endmembers from the subsets 222 and/or the endmembers from the whole data set 221 can then be combined to determine the significant components spectra 223. Abundance map 224 can then be derived from significant component spectra 223. In certain embodiments abundance map 224 can be a quantitative spatial distribution of all the detected significant components 223.

FIG. 3 is a diagram illustrating embodiments of unsupervised unmixing algorithms according to some examples of the disclosed subject matter. In particular, FIG. 3 illustrates an ideal or theoretical absorption spectra of oxy-deoxy hemoglobin 300 and the background tissue absorption compared to different unsupervised spectral unmixing processes or algorithms 310-360 processing test data, such as test multi-spectral photoacoustic image data. SVD 310 and PCA 320 are unsupervised dimension reduction approaches in machine learning. Both SVD 310 and PCA 320 rely on the source components being uncorrelated and orthogonal. In PCA 320, the goal can be to reduce the correlated observed variables. ICA 330 and RICA 340, on the other hand, assume that the observation data can be a superimposition of a number of stochastically independent processes. ICA 330 relies on the source components being maximally independent and non-Gaussian. The non-Gaussianity of the source data can make ICA 330 more powerful than PCA 320 in some non-limiting embodiments. RICA 340 takes ICA 330 a step further by adding a reconstruction cost to the cost function of the standard ICA approach.

In contrast to the previous algorithms 310-340, SF 350 cannot explicitly be based on a model of data distribution. SF 350 can be based on optimization of sparsity of the features distribution and/or can be formulated by implementing an uncomplicated code. In some non-limiting embodiments, SF 350 can be computationally expensive. In contrast, NNMF 360 can be a linear mixture model based on using non-negative matrices. In other words, NNMF 360 can assume that the source data can be non-negative, making NNMF 360 compatible with PA imaging in which all pixel values are either zero or positive. Imposing a positivity condition, similar to NNMF 360, can help to enhance the convergency of the optimization algorithm used for the unmixing problem. In addition, or as an alternative, by imposing the nonnegativity constraints NNMF 360 can learn or process a parts-based representation of the data, allowing a whole image to be formed as a combination of additive components. Processing multispectral photoacoustic image data with NNMF 360 can result in a non-negative matrix X of mixed observation data that can be factorized into a source and mixing coefficient matrices.

As shown in FIG. 3, NNMF 360 can have a better sensitivity and specificity than SVD 310, PCA 320, ICA 330, RICA 340, and/or SF 350. NNMF 360 can also be most similar to the ideal absorption spectral of oxy-deoxy hemoglobin.

Further, below Table A shows the correlation values evaluated between the ideal oxy and deoxy HB spectra and the extracted prominent spectra found by using the different unsupervised unmixing algorithms.

**TABLE A**

|  | SVD | PCA | ICA | RICA | SF | NNMF |
|---|---|---|---|---|---|---|
| **Oxy HB** | 0.9676 | -0.9721 | 0.1471 | 0.9811 | 0.9869 | 1 |
| **Deoxy HB** | 0.8993 | 0.9698 | -0.1180 | -0.9229 | 0.9775 | 1 |

As shown in Table A, NNMF 360 has the highest positive correlation. The high correlation can demonstrate the ability of unsupervised spectral unmixing process or algorithm NNMF 360 to detect both endogenous (oxy, deoxy hemoglobin) and exogenous characteristics (contrast agents) spectra.

Despite the advantages provided by NNMF 360, in certain non-limiting embodiments any other unsupervised spectral unmixing process or algorithm can be used. For example, certain non-limiting embodiments can select an unsupervised spectral unmixing process or algorithm without a non-negative constraint. The non-negative constraint can be computationally expensive to implement, and each component can be estimated only up to a multiple scale factor. To lower the number of computer or processing resources, some non-limiting embodiments can use SVD 310, PCA 320, ICA 330, RICA 340, SF 350, or any other known unsupervised algorithm.

FIG. 4 is a diagram illustrating an unsupervised unmixing system or method according to some examples of the disclosed subject matter. For input 410, a PA imaging system 411 can be used to image two-dimensional region across the entire wavelength NIR range of 680 - 970 nm with a step size of 5 nm. In other words, 59 two-dimensional images of the same or similar region of interest can be captured. The captured images can be referred to as multi-spectral photoacoustic image data. For pre-processing 420, the multi-spectral photoacoustic image data can undergo data correction and/or data reduction. In certain non-limiting embodiments pre-processing 420 can include determining the number of significant components which are above the noise level from the multi-spectral photoacoustic image data. The noise level and the number of significant components can be determined using one or more eigenvalue algorithms. While the method or system shown in FIG. 4 utilizes unsupervised spectral unmixing process or algorithm, the pre-processing can be supervised or unsupervised. For example, if the pre-processing is supervised a user can intervene by changing or adjusting the number of significant components.

NNMF 430 can then be used to detect tissue chromophores from the multi-spectral photoacoustic image data. The detecting can be based on the number of significant components above the noise floor as determined during pre-processing 420. NNMF 430 can be represented using the following equation: $X \approx WS$, where $X$ is the matrix containing the mixed multi spectral observations or the multi-spectral photoacoustic image data. $X$ can be factorized into a $n \times r$

matrix *W* and a *r x* m matrix *S,* where *W* represents abundance distribution component values and *S* represents the main spectral curves. The number of prominent component sources *r* can be a hyperparameter, which can be smaller than *n* or *m*. *r* can also be referred to as the number of significant components. Using NNMF 430 can result in a dimensional reduction of the original mixed data, also referred to as multi-spectral photoacoustic image data, into endmembers and their respective abundance per each pixel.

As discussed above, NNMF 430 unmixed the multi-spectral photoacoustic image data using an optimization iterative approach. Each iteration of the unsupervised spectral unmixing process or algorithm can minimize the following cost function: $\frac{min}{w,s} \frac{1}{2} \|X - WS\|_F^2, W \geq 0, S \geq 0$, where X represents the mixed observations, *W* represents the abundance maps, and S represents the source spectra. *W* and *S* are restricted to non-negative matrices. Matrices *W* and *S* are iteratively obtained using the above cost function to minimize the root mean squared residual. As such, the cost function can evaluate the quality of the approximate factorization. Since no elements of the above equation are negative, the unsupervised spectral unmixing process or algorithm can be a process that generates the original data by linear combinations of the prominent components, meaning that the tissue chromophores are detected based on the number of significant components from the multi-spectral photoacoustic image data. At the end of the iterative minimization, NNMF 430 can provide main spectral curves in *S.*

In certain non-limiting embodiments, the unsupervised spectral unmixing process or algorithm, such as NNMF 430, can include clustering and windowing 440 to determine the prominent tissue chromophores present in the multi-spectral photoacoustic image data. Clustering process include grouping of the spectra which are similar and differentiating the spectra which are easily distinguishable after applying the first iteration of NNMF algorithm. The spectra which are similar will have higher correlation and the distinguishable will have lower correlation values. Using clustering 440, the unsupervised spectral unmixing process or algorithm can find one or more significant components, in a given wavelength range or step size, which are having lower correlation values. To further investigate the groups of highly correlated spectra and thus differentiate the compounds, a windowing approach is implemented. In this approach, multiple subsets of spectra can be created from the original data set (X). Employing windowing 440 allows for searching the significant components 450 in each individual subset, rather than searching the data as a whole. The resulting subsets of spectra can reduce the number of wavelength and observations. For each subset, significant component above the noise floor and the NNMF can be estimated. After each subset is searched, the detected tissue chromophores from each subsets and the tissue chromophores detected earlier from the clustering can be combined. Combining the detected tissue chromophores for example, can include superimposing or overlapping the spectra or any other method of combination. Clustering and windowing 440 can help to increase the sensitivity of the unsupervised spectral unmixing process or algorithm.

Using pre-processing 420, NNMF 430, and/or clustering and windowing 440, tissue chromophores for one or more components 450 can be detected. Components 450 can be biomarkers, such as melanin, water, collagen, lipids, hemoglobin, oxyhemoglobin, deoxyhemoglobin, and myoglobin. At the end of the iterative minimization, the unsupervised spectral unmixing process or algorithm, such as NNMF 430, can provide main spectral curve in *S* and/or abundance distribution component values *W* reshaped into original dimension image masks. Each pixel of the masks can quantify the presence of the different prominent components. In FIG. 4 source spectra 460 illustrates the outputted main spectral curve, while abundance maps 470 illustrate three different abundance distribution components.

FIG. 5 is a diagram illustrating pre-processing according to some examples of the disclosed subject matter. In particular, FIG. 5 illustrates the input matrix processed by the unsupervised spectral unmixing process or algorithm, such as NNMF 430. As shown in FIG. 5, multi-spectral PA images 500, which includes original data set 2D+λ, can be pre-processed, for example, using data correction 510 and data reduction 520. Data correction 510 can include a noise removal or reduction step that uses a Gaussian filter having a kernel of 5x5 pixels. Data reduction 520 can include a reduction of the number of observations to limit the computational cost. By using a squared ROI of 4x4 pixels to average the pixels into the region of interest (ROI), the number of pixels per image can be reduced. In some non-limiting embodiments data correction 510 can be within a given two-dimensional image, while data reduction or removal 520 can be between two or more two-dimensional images.

In some non-limiting embodiments, the mixed spectra can be structured into an *n x m* matrix 530. The *n* rows of matrix 530 can represent the number of observations or pixels, also referred to as the region of interest, while *m* columns represent the number of variables per object or different wavelengths. In other words, the received or acquired data can be organized into matrix 530, where each column refers to a vectorized PA image obtained at a specific wavelength. Mixed spectra 540 can illustrate a single row of matrix 530, further stressing the need for using a mixing algorithm to evaluate the mixed data.

FIG. 6 is a diagram illustrating abundance maps for supervised and unsupervised unmixing algorithms according to some examples of the disclosed subject matter. In particular, FIG. 6 illustrates abundance maps for oxyhemoglobin 630, deoxyhemoglobin 640, and ICG 650 using a supervised spectral unmixing algorithm 620 and unsupervised spectral unmixing process or algorithm 610. The abundance maps illustrated the detected tissue chromophores for oxyhemoglobin 630, deoxyhemoglobin 640, and/or ICG 650. In addition, one or more of the abundance maps can be composed of overlapped or superimposed image clusters. As shown in FIG. 6, unsupervised spectral unmixing process or algorithm 620 results in a more sensitive and specific abundance map compared to supervised spectral unmixing algorithm 610. Unsupervised spectral unmixing process or algorithm 620 can account for variations in the spectral curve when a molecule is in a different environment or condition. For example, the spectral characteristics of many dyes, such as ICG, can change in living tissues. Further, the theoretical absorption spectra of the tissue chromophores for pathological conditions, diseases, or health conditions can also change characteristics. Unsupervised spectral unmixing process or algorithm 620 can account for this changed absorption spectra.

FIG. 7 is a diagram illustrating a component spectra according to some examples of the disclosed subject matter. In particular, FIG. 7 illustrates component spectra associated with a determined significant number of components from the multi-spectral photoacoustic image data. In some non-limiting embodiments, the significant number of components 710 can be determined by the user. The user, for example, can select three components based on a-priori information, such as a user defined noise floor level. The tissue chromophores can then be detected based on the user selected significant number of components. In some non-limiting embodiments the significant number of components can be referred to as a threshold or prominent number of components. The resulting component spectra 720 only includes the three selected components. When compared to theoretical spectra 730, component spectra 720 does not illustrate many of the potentially significant components.

In some other non-limiting embodiments, therefore, the significant number of components can be determined using machine learning. For example, the number of significant components can be based on the noise floor of the multi-spectral photoacoustic image data. The noise floor and/or the number of significant can be determined, in certain non-limiting embodiments, using an eigenvalue algorithm or equation. As shown in FIG. 7, using an eigenvalue algorithm or equation can determine a higher number of significant components 711, with the resulting component spectra 721 illustrating information related to seven different components. This can allow a user to observe possible correlations between components that were not previously known to the user. For example, the eigenvalue algorithm or equation can determine that a lower wavelength range should be processed by the unsupervised spectral unmixing process or algorithm, leading to the detection of one or more significant components. Even though the user may not previously believe that a component included within the wavelength range, such as lipids, should be included as a significant component, using the eigenvalue algorithm or equation can allow a user to view the resulting lipids spectra.

FIG. 8 is a flow diagram of a method or process according to some examples of the disclosed subject matter. In particular, the method or process can be performed by any apparatus that includes a processor, memory, and/or a graphical user interface. The apparatus can be a computer, cloud computer, mobile device, server, medical imaging device, PA imaging device, ultrasound imaging device, or any other device that includes a processor, memory, and/or graphical user interface. In some non-limiting embodiments PA imaging and ultrasound imaging can be performed by a single device.

In step 810, the PA imaging method includes receiving the multi-spectral PA image data from a photoacoustic imaging system. The multi-spectral PA image data is pre-processed as shown in step 820. The pre-processing includes determining a number of significant components above a noise floor of the multi-spectral photoacoustic image data, as shown in step 830. At least one of the number of significant components and/or noise floor can be determined using an eigenvalue algorithm. In some non-limiting embodiments, the significant number of components comprises melanin, oxyhemoglobin, deoxyhemoglobin, lipids myoglobin and water. The pre-processing of the multi-spectral PA image data can also include at least one of data correction or data reduction. The data correction can include a Gaussian filter, and the data reduction can include using a squared region of interest.

In step 840, the method includes detecting tissue chromophores based on the significant number of components from the multi-spectral photoacoustic image data using an unsupervised spectral unmixing process or algorithm. The unsupervised spectral unmixing process includes clustering and windowing of the multi-spectral photoacoustic image data. The unsupervised spectral unmixing process or algorithm, for example, can include nonnegative matrix factorization.

The nonnegative matrix factorization can be represented by $\frac{min}{w,s} \ \frac{1}{2} \ \|X - WS\|_F^2$, $W \geq 0$, $S \geq 0$, where X represents the mixed observations, $W$ represents the abundance maps, $S$ represents main spectral curves. In other examples, the unsupervised spectral unmixing process or algorithm can comprise principal component analysis, independent component analysis, reconstruction independent component analysis, or sparse filtering.

In step 850, the detected tissue chromophores are displayed in an abundance map. In step 860, a component spectra with the determined number of components can be displayed from the multi-spectral photoacoustic image data. The component spectra can represent a pure molecule absorption spectrum extracted from the multi-spectral photoacoustic image data. The multi-spectral photoacoustic image data, for example, can be received at wavelengths between 680 and 970 nanometers.

FIG. 9 is an example of an apparatus according to some non-limiting embodiments of the disclosed subject matter. In particular, FIG. 9 illustrates an apparatus 910, such as a computer, mobile device, server, medical imaging device, PA imaging device, ultrasound system or device, or any other device that includes a processor 911, memory 912, and/or graphical user interface 914. In one embodiment the apparatus can be an ultrasound system, for example, a portable point-of-care ultrasound, which can be hand held, portable, or cartbased. It should be understood that each feature of FIGS. 1-9, and any combination thereof, can be implemented by an apparatus or an ultrasound and photoacoustic system, using various hardware, software, firmware, and/or one or more processors or circuitry, in connection with various different embodiments of the disclosed subject matter.

The apparatus includes at least one processor 911 or control unit. At least one memory is also provided in each apparatus, indicated as 912. Memory 912 includes computer program instructions or computer code contained therein, which instructions or code can be executed by the processor. The system can also include networked components communicating over a local network, a wide area network, wirelessly and/or wired, or by any other coupling that allows communication of data from one system component to another.

In certain non-limiting embodiments one or more transceivers 913 can be provided. The one or more transceivers 913 can receive signals from transducer probe 916, also referred to as transducer, which transmits and/or receives sound waves to and from the subject or body being examined. Transducer probe 916 can transmit the signal to apparatus 910 via a wireless or wired communication.

Transducer probe 916 can be able to transmit sound waves of various frequencies and receive echo signals. The sound waves, for example, can range from a low bandwidth frequency of 3 Megahertz (MHz) to as high frequency of 71 MHz. Other non-limiting embodiments can use any other soundwave frequency. Higher frequencies can allow for the imaging of superficial structures, while lower frequencies can allow for the deeper tissue imaging with each typically providing different resolutions. Transducer probe 916 can in some non-limiting embodiments also include a beamformer.

In some non-limiting embodiments, transducer probe 916 can be a single element or a multi-element transducer that is moved to sweep the transducer over a range of beam angles. Transducer probe 916 can use either wired or wireless communication to send and/or receive information to apparatus 910. The transmitted information can be saved in memory 912, or in any other external memory or database.

The ultrasound system can also include any other component not shown in FIG. 10, such as an analog front-end that includes, for example, a low noise amplifier (LNA), a voltage controlled attenuator (VCAT), an analog to digital converter, and/or a beamformer receiver. Once the analog sound signal is received by the probe, it can be amplified on the front end of the ultrasound system and converted into a digital format using any known analog to digital converter. Once converted into digital form, the signal can be transmitted to apparatus 910. Apparatus 910 can include or be connected to display 914, which can display the received digital information.

In certain non-limiting embodiments, display 914 can be located in a separate apparatus from apparatus or ultrasound machine 910. In yet another example, instead of a display the apparatus can include a projector capable of projecting the image onto an external display or screen, or can include active eyeglasses or headset that can be worn by the operator of the ultrasound system in order to view the displayed data.

In some non-limiting embodiments, apparatus 910 can be a medical imaging device, such as an ultrasound system, configured to carry out the embodiments described above in relation to FIGS. 1-8. In certain non-limiting embodiments, at least one memory including computer program code can be configured to, when executed by the at least one processor, cause the apparatus to perform any or all of the processes described herein. Processor 911 can be embodied by any computational or data processing device, such as a central processing unit (CPU), digital signal processor (DSP), application specific integrated circuit (ASIC), programmable logic devices (PLDs), field programmable gate arrays (FPGAs), input/output (I/O) circuitry, digitally enhanced circuits, or comparable device, or any combination thereof. In one example, the ASIC described in U.S. Patent No. 8,213,467 can be used. U.S. Patent No. 8,213,467 is hereby incorporated by reference in its entirety. The processors can be implemented as a single controller, or a plurality of controllers or processors.

The ultrasound system can also include a system control panel 915. System control panel 915, such as the tactile gain control used in, for example, can include the user interface, touchpad, or touchscreen used to adjust the near, middle, and far middle gain control. The system control panel, can alternatively or in addition to, include other controls for adjusting or changing various settings of the ultrasound system.

For firmware or software, the implementation can include modules or a unit of at least one chip set (for example, including procedures and/or functions). Memory 912 can independently be any suitable storage device, such as a non-transitory computer-readable medium, a hard disk drive (HDD), random access memory (RAM), flash memory, or other suitable memory. The memories can be combined on a single integrated circuit with a processor, or can be separate therefrom. Furthermore, the computer program instructions can be stored in the memory and be processed by the processors, and can be any suitable form of computer program code, for example, a compiled or interpreted computer program written in any suitable programming language. For example, in certain non-limiting embodiments, a non-transitory computer-readable medium can be encoded with computer instructions or one or more computer programs (such as added or updated software routine, applet or macro) that, when executed in hardware, can perform a process such as one of the processes described herein. Computer programs can be coded by a programming language, which can be a highlevel programming language, such as objective-C, C, C++, C#, Java, etc., or a low-level programming language, such as a machine language, or assembler. Alternatively, certain non-limiting embodiments can be performed entirely in hardware.

In certain non-limiting embodiments FIG. 9 can include a laser 917. Laser 917 can be used as part of the PA imaging system or apparatus. In particular, laser 917 can deliver nonionizing pulses into biological tissue. Laser 917 can be absorbed by the tissue, causing expansion and the emission of ultrasonic waves detected by transducer 916. In some non-limiting embodiments the laser can be a nanosecond pulsed laser capable of emitting 680 - 2000 nanometer wavelengths.

The above embodiments provide significant technical improvements and advantages to the apparatus itself and for PA imaging in general. The use of an unsupervised spectral unmixing process or algorithm in PA imaging can provide improved tissue chromophores detection. The use of clustering and windowing as part of the unsupervised spectral unmixing process or algorithm, as well as the determining of the significant components, provide additional significant technical improvements. The abundance maps shown in FIG. 6 illustrate the technical improvements in PA imaging provided for by the unsupervised spectral unmixing process or algorithm, as opposed to traditional supervised spectral unmixing algorithms.

In addition to the above significant technical improvements in PA imaging, the disclosed embodiments also provide advantages to the apparatus itself. For example, removing all user interaction can reduce the number of processor, memory, and/or network resources needed to process the multi-spectral photoacoustic image data. Outputting improved abundance maps and component spectra can also help with the accurate determining of disease or medical conditions, thereby limiting the need for further processing of multi-spectral photoacoustic image data.

The features, structures, or characteristics of certain embodiments described throughout this specification can be combined in any suitable manner in one or more embodiments. For example, the usage of the phrases "certain embodiments," "some embodiments," "other embodiments," or other similar language, throughout this specification refers to the fact that a particular feature, structure, or characteristic described in connection with the embodiment can be included in at least one embodiment of the disclosed subject matter. Thus, appearance of the phrases "in certain embodiments," "in some embodiments," "in other embodiments," or other similar language, throughout this specification does not necessarily refer to the same group of embodiments, and the described features, structures, or characteristics can be combined in any suitable manner in one or more embodiments.

One having ordinary skill in the art will readily understand that the disclosed subject matter as discussed above can be practiced with procedures in a different order, and/or with hardware elements in configurations which are different from those disclosed. Therefore, although the disclosed subject matter has been described based upon these embodiments, it would be apparent to those of skill in the art that certain modifications, variations, and alternative constructions would be apparent, while remaining within the scope of the invention as defined by the appended claims.

**Claims**

1. A photoacoustic imaging method comprising:

   receiving (810) multi-spectral photoacoustic image data from a photoacoustic imaging system;
   pre-processing (820) the multi-spectral photoacoustic image data, wherein the pre-processing comprises determining (830) a number of significant components (216) above a noise floor (215) of the multi-spectral photoacoustic image data; and
   detecting (840) tissue chromophores based on the number of significant components (216) from the multi-spectral photoacoustic image data using an unsupervised spectral unmixing process (620);
   **characterised by** displaying (850) the detected tissue chromophores in an abundance map (224),
   wherein the unsupervised spectral unmixing process (620) comprises clustering and windowing (440) of the multi-spectral photoacoustic image data.

2. The method according to claim 1, further comprising:
   displaying a component spectra (223) with the determined number of components from the multi-spectral photoacoustic image data.

3. The method according to claim 2, wherein the component spectra (720) represents a pure molecule absorption spectrum extracted from the multi-spectral photoacoustic image data.

4. The method according to any of claims 1-3, wherein the significant number of components (216) comprises melanin, oxyhemoglobin, deoxyhemoglobin, lipids myoglobin and water.

5. The method according to any of claims 1-4, wherein the unsupervised spectral unmixing process (620) comprises nonnegative matrix factorization.

6. The method according to claim 5, wherein the nonnegative matrix factorization is represented by

$$\frac{min}{w,s} \; \frac{1}{2} \; \|X - WS\|_F^2$$

, $W \geq 0$, $S \geq 0$, where W represents abundance distribution component values, S represents main spectral curves, and X represents the multispectral observations.

7. The method according to any of claims 1-6, wherein the unsupervised spectral unmixing process (620) comprises principal component analysis, independent component analysis, reconstruction independent component analysis, or sparse filtering.

8. The method according to any of claims 1-7, wherein at least one of the number of significant components (216) or noise floor (215) is determined using an eigenvalue algorithm.

9. The method according to any of claims 1-8, wherein the clustering and windowing (440) comprises:

dividing the multi-spectral photoacoustic image data into one or more subsets; and
searching for the number of significant components (216) in the one or more subsets.

10. The method according to any of claims 1-9, wherein the pre-processing (820) of the multi-spectral photoacoustic image data further comprises at least one of data correction (212) or data reduction (213), wherein the data correction (212) comprises a Gaussian filter, and wherein the data reduction (213) comprises using a squared region of interest of 4x4 pixels.

11. The method according to any of claims 1-10, wherein the multi-spectral photoacoustic image data is received at wavelengths between 680 and 970 nanometers.

12. A photoacoustic imaging apparatus (910) comprising:

at least one memory (912) comprising computer program code;
at least one processor (911);
wherein the at least one memory (912) comprising the computer program code are configured, with the at least one processor (911), to cause the photoacoustic imaging apparatus (910) at least to perform the method of any of claims 1-11.

13. The photoacoustic imaging apparatus (910) according to claim 12, wherein the at least one memory (912) comprising the computer program code are configured, with the at least one processor (911), to cause the apparatus (910) at least to:
determine a disease or medical condition based on at least one of the abundance map (224) or a component spectra (223).

14. A non-transitory computer-readable medium encoding instructions that, when executed on a photoacoustic imaging apparatus (910), perform a process according to the method of any of claims 1-11.

15. A computer program product encoding instructions for performing, when run on a photoacoustic imaging apparatus (910), a process according to the method of any of claims 1-11.

**Patentansprüche**

1. Fotoakustisches Bildgebungsverfahren, umfassend:

Empfangen (810) von multispektralen fotoakustischen Bilddaten von einem fotoakustischen Bildgebungssystem;

Vorverarbeiten (820) der multispektralen fotoakustischen Bilddaten, wobei das Vorverarbeiten umfasst, eine Anzahl von signifikanten Komponenten (216) über einem Grundrauschen (215) der multispektralen fotoakustischen Bilddaten zu bestimmen (830); und

Erfassen (840) von Gewebechromophoren basierend auf der Anzahl von signifikanten Komponenten (216) aus den multispektralen fotoakustischen Bilddaten unter Verwendung eines unüberwachten Spektralentmischungsprozesses (620);

**gekennzeichnet durch** Anzeigen (850) der erfassten Gewebechromophore in einer Abundanzkarte (224);

wobei der unüberwachte Spektralentmischungsprozess (620) umfasst, die multispektralen fotoakustischen Bilddaten zu clustern und zu fenstern (440).

2. Verfahren nach Anspruch 1, weiter umfassend:
Anzeigen eines Komponentenspektrums (223) mit der bestimmten Anzahl von Komponenten aus den multispektralen fotoakustischen Bilddaten.

3. Verfahren nach Anspruch 2, wobei das Komponentenspektrum (720) ein reines Molekülabsorptionsspektrum darstellt, das aus den multispektralen fotoakustischen Bilddaten extrahiert wird.

4. Verfahren nach einem der Ansprüche 1-3, wobei die signifikante Anzahl von Komponenten (216) Melanin, Oxyhämoglobin, Desoxyhämoglobin, Myoglobin von Lipiden und Wasser umfasst.

5. Verfahren nach einem der Ansprüche 1-4, wobei der unüberwachte Spektralentmischungsprozess (620) nichtnegative Matrizenfaktorisierung umfasst.

6. Verfahren nach Anspruch 5, wobei die nichtnegative Matrizenfaktorisierung durch $\frac{min}{w,s} \frac{1}{2} \ ||X - WS||_F^2$ , $W \geq 0$, $S \geq 0$, dargestellt wird, wo W Abundanzverteilungskomponentenwerte darstellt, S Hauptspektralkurven darstellt und X die multispektralen Beobachtungen darstellt.

7. Verfahren nach einem der Ansprüche 1-6, wobei der unüberwachte Spektralentmischungsprozess (620) Hauptkomponentenanalyse, Unabhängigkeitsanalyse, rekonstruktive Unabhängigkeitsanalyse (Reconstruction Independent Component Analysis) oder Sparse Filtering umfasst.

8. Verfahren nach einem der Ansprüche 1-7, wobei mindestens eines von der Anzahl von signifikanten Komponenten (216) oder dem Grundrauschen (215) unter Verwendung eines Eigenwert-Algorithmus bestimmt wird.

9. Verfahren nach einem der Ansprüche 1-8, wobei das Clustern und Fenstern (440) umfasst:
Aufteilen der multispektralen fotoakustischen Bilddaten in einen oder mehrere Teilsätze; und Suchen nach der Anzahl von signifikanten Komponenten (216) in dem einen oder den mehreren Teilsätzen.

10. Verfahren nach einem der Ansprüche 1-9, wobei das Vorverarbeiten (820) der multispektralen fotoakustischen Bilddaten weiter mindestens eines von Datenkorrektur (212) oder Datenverringerung (213) umfasst, wobei die Datenkorrektur (212) einen Gaußschen Filter umfasst und wobei die Datenverringerung (213) das Verwenden eines quadratischen Bereichs von Interesse von 4x4 Pixel umfasst.

11. Verfahren nach einem der Ansprüche 1-10, wobei die multispektralen fotoakustischen Bilddaten bei Wellenlängen zwischen 680 und 970 Nanometern empfangen werden.

12. Fotoakustische Bildgebungseinrichtung (910), umfassend: mindestens einen Speicher (912) der Computerprogrammcode umfasst;

mindestens einen Prozessor (911);

wobei der mindestens eine Speicher (912), umfassend den Computerprogrammcode, konfiguriert sind, um mit dem mindestens einen Prozessor (911) die fotoakustische Bildgebungseinrichtung (910) zu veranlassen, das Verfahren nach einem der Ansprüche 1-11 durchzuführen.

13. Fotoakustische Bildgebungseinrichtung (910) nach Anspruch 12, wobei der mindestens eine Speicher (912), umfassend den Computerprogrammcode, konfiguriert sind, um mit dem mindestens einen Prozessor (911) die Einrichtung (910) zu veranlassen zum:
Bestimmen einer Krankheit oder eines medizinischen Zustands basierend auf mindestens einem von der Abundanzkarte (224) oder einem Komponentenspektrum (223).

14. Nichtflüchtiges computerlesbares Medium, das Anweisungen codiert, die, wenn sie auf einer fotoakustischen Bildgebungseinrichtung (910) ausgeführt werden, einen Prozess nach dem Verfahren nach einem der Ansprüche 1-11 durchführen.

15. Computerprogrammprodukt, das Anweisungen zum Durchführen, wenn sie auf einer fotoakustischen Bildgebungseinrichtung (910) laufen, eines Prozesses nach dem Verfahren nach einem der Ansprüche 1-11 codiert.

**Revendications**

1. Procédé d'imagerie photoacoustique comprenant :

   la réception (810) de données d'image photoacoustique multispectrale provenant d'un système d'imagerie photoacoustique ;
   le prétraitement (820) des données d'image photoacoustique multispectrale, dans lequel le prétraitement comprend la détermination (830) d'un nombre de composantes significatives (216) au-dessus d'un plancher de bruit (215) des données d'image photoacoustique multispectrale ; et
   la détection (840) de chromophores tissulaires sur la base du nombre de composantes significatives (216) des données d'image photoacoustique multispectrale en utilisant un processus de démélange spectral non supervisé (620) ;
   **caractérisé par** l'affichage (850) des chromophores tissulaires détectés dans une carte d'abondance (224), dans lequel le processus de démélange spectral non supervisé (620) comprend le regroupement et le fenêtrage (440) des données d'image photoacoustique multispectrale.

2. Procédé selon la revendication 1, comprenant en outre :
   l'affichage d'un spectre de composantes (223) avec le nombre déterminé de composantes à partir des données d'image photoacoustique multispectrale.

3. Procédé selon la revendication 2, dans lequel le spectre de composantes (720) représente un spectre d'absorption de molécule pure extrait des données d'image photoacoustique multispectrale.

4. Procédé selon l'une quelconque des revendications 1-3, dans lequel le nombre de composantes significatives (216) comprend la mélanine, l'oxyhémoglobine, la désoxyhémoglobine, les lipides, la myoglobine et l'eau.

5. Procédé selon l'une quelconque des revendications 1-4, dans lequel le processus de démélange spectral non supervisé (620) comprend une factorisation matricielle non négative.

6. Procédé selon la revendication 5, dans lequel la factorisation matricielle non négative est représentée par

   $$\frac{min}{w,s}\frac{1}{2}||X - WS||_F^2$$ , $W \geq 0$, $S \geq 0$, où W représente des valeurs de composantes de distribution d'abondance, S représente des courbes spectrales principales, et X représente les observations multispectrales.

7. Procédé selon l'une quelconque des revendications 1-6, dans lequel le processus de démélange spectral non supervisé (620) comprend une analyse en composantes principales, une analyse en composantes indépendantes, une analyse en composantes indépendantes par reconstruction ou un filtrage clairsemé.

8. Procédé selon l'une quelconque des revendications 1-7, dans lequel au moins l'un parmi le nombre de composantes significatives (216) ou le plancher de bruit (215) est déterminé en utilisant un algorithme de valeurs propres.

9. Procédé selon l'une quelconque des revendications 1-8, dans lequel le regroupement et le fenêtrage (440) comprennent :
   la division des données d'image photoacoustique multispectrale en un ou plusieurs sous-ensembles ; et la recherche du nombre de composantes significatives (216) dans les un ou plusieurs sous-ensembles.

**10.** Procédé selon l'une quelconque des revendications 1-9, dans lequel le prétraitement (820) des données d'image photoacoustique multispectrale comprend en outre au moins l'une parmi la correction de données (212) ou la réduction de données (213), dans lequel la correction de données (212) comprend un filtre de Gauss, et dans lequel la réduction de données (213) comprend l'utilisation d'une région d'intérêt quadrillée de 4x4 pixels.

**11.** Procédé selon l'une quelconque des revendications 1-10, dans lequel les données d'image photoacoustique multispectrale sont reçues à des longueurs d'onde comprises entre 680 et 970 nanomètres.

**12.** Appareil d'imagerie photoacoustique (910) comprenant :

au moins une mémoire (912) comprenant un code de programme informatique ;
au moins un processeur (911) ;
dans lequel la au moins une mémoire (912) comprenant le code de programme informatique est configurée, avec le au moins un processeur (911), pour amener l'appareil d'imagerie photoacoustique (910) à au moins effectuer le procédé selon l'une quelconque des revendications 1-11.

**13.** Appareil d'imagerie photoacoustique (910) selon la revendication 12, dans lequel la au moins une mémoire (912) comprenant le code de programme informatique est configurée, avec le au moins un processeur (911), pour amener l'appareil (910) à au moins :
déterminer une maladie ou un trouble pathologique sur la base d'au moins l'un parmi la carte d'abondance (224) ou un spectre de composantes (223).

**14.** Support non transitoire lisible par ordinateur codant des instructions qui, lorsqu'elles sont exécutées sur un appareil d'imagerie photoacoustique (910), effectuent un processus selon le procédé de l'une quelconque des revendications 1-11.

**15.** Produit de programme informatique codant des instructions pour effectuer, lorsqu'elles sont exécutées sur un appareil d'imagerie photoacoustique (910), un processus selon le procédé de l'une quelconque des revendications 1-11.

FIG. 1

| Input | **210** | Multi Spectral PA Images | |
|---|---|---|---|
| Pre-processing steps **211** | Background Removal | Data Correction | Data Reduction **213** |
| Linear Mixing Model X: Matrix of the mixed observed data | **212** | Mixed Observations **214** | |
| Eigenvalues Evaluation | **215** Noise Floor | Number of Significant Components **216** | |
| Unsupervised Unmixing ML Algorithms | **217** PCA, ICA, RICA SF, NNMF | **219** | |
| First Outcomes | **218** Endmembers | Abundance Maps | |
| Clustering | **220** Similar Spectra | Easily Distinguishable Spectra **221** | |
| Windowing | Subsets **222** | | |
| Final Outcomes: Combining Endmembers from the Subsets and Endmembers from the whole Data Set* | **223** Significant Components Spectra | | |
| Final Outcomes: Quantitative Spatial Distribution of all the detected Significant Components | **224** Abundance Maps | | |

FIG. 2

FIG. 3

FIG. 3 Continued

EP 4 125 558 B1

FIG. 4

EP 4 125 558 B1

FIG. 4
Continued

FIG. 5

630 $Hb_{OXY}$
640 $Hb_{DEOXY}$
650 ICG
610 SUPERVISED
620 BLIND

FIG. 6

EP 4 125 558 B1

| EIGENVALUES | Prominent Tissue Chromophores Absorption Curves | |
|---|---|---|
| # OF SIGNIFICANT COMPONENTS (ENDMEMBERS) ABOVE THE NOISE FLOOR | NNMF–ESTIMATED | THEORETICAL |

FIG. 7

EP 4 125 558 B1

810 — receiving multi-spectral photoacoustic image data

820 — pre-processing the multi-spectral photoacoustic image data

830 — determining a significant number of components above a noise floor

840 — detecting tissue chromophores using an unsupervised spectral unmixing algorithm including clustering and windowing

850 — displaying the detected issue chromophores in an abundance map

860 — displaying a component spectra with the determine number of components

870 — determining a disease or medical condition

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015101411 A1

- US 8213467 B

**Non-patent literature cited in the description**

- **ARABUL M.U. et al.** Unmixing multi-spectral photoacoustic sources in human carotid plaques using non-negative independent component analysis. *PHOTOACOUSTICS,* 01 September 2019, vol. 15

- **GLATZ URGEN et al.** Blind source unmixing in multi-spectral optoacoustic tomography References and links. *Nat. Biotechnol. Rev. Sci. Instrum. Nat. Methods Opt. Lett. Phys. Rev. Lett. Chem. Rev. Opt. Express Opt. Lett. Appl. Phys. Lett. Clin. Chem. J. Biomed. Opt. OPTICS EXPRESS Opt. Lett. Nat. Photonics Opt. Express Med. Phys. IEEE Trans. Med. Imaging An,* 01 January 2011